(19) Europäisches Patentamt

European Patent Office

Office européen des brevets

(11) **EP 1 312 850 B1**

(12) **EUROPÄISCHE PATENTSCHRIFT**

(45) Veröffentlichungstag und Bekanntmachung des Hinweises auf die Patenterteilung:
**02.11.2006 Patentblatt 2006/44**

(51) Int Cl.:
*F16M 11/06* (2006.01) *G02B 7/00* (2006.01)

(21) Anmeldenummer: **02020706.4**

(22) Anmeldetag: **14.09.2002**

(54) **Vorrichtung und Verfahren für wenigstens teilweisen Ausgleich eines Last-Drehmoments**

Device and method for balancing at least partiaily a load torque

Dispositif et procédé pour équilibrer au moins partiellement un couple de charge

(84) Benannte Vertragsstaaten:
**CH DE FR GB LI**

(30) Priorität: **19.11.2001 DE 10156318**

(43) Veröffentlichungstag der Anmeldung:
**21.05.2003 Patentblatt 2003/21**

(73) Patentinhaber: **Carl Zeiss Surgical GmbH**
**73447 Oberkochen (DE)**

(72) Erfinder:
• **Gaertner, Hartmut**
**73447 Oberkochen (DE)**

• **Zimmermann, Fritz**
**73457 Esslingen (DE)**
• **Strauss, Wolfgang**
**89195 Staig (DE)**

(74) Vertreter: **Gauss, Nikolai et al**
**c/o Carl Zeiss AG**
**Patentabteilung**
**73446 Oberkochen (DE)**

(56) Entgegenhaltungen:
**DE-A- 2 952 660      DE-A- 3 714 637**
**DE-A- 3 739 080      US-A- 5 642 220**

EP 1 312 850 B1

**Beschreibung**

Die Erfindung betrifft eine Vorrichtung zum wenigstens teilweisen Ausgleich eines Last- Drehmoments, welches an einer Masseeinheit, insbesondere einem Operationsmikroskop angreift, das an einer Haltevorrichtung drehbar gelagert ist, mit einer Einheit zur Erzeugung von Linearkraft und einer Wandlereinheit, welche die Linearkraft in ein Drehmoment wandelt, bei der eine einstellbare Kopplungseinheit zur Übertragung des Drehmoments zwischen der Wandlereinheit und der Masseeinheit vorgesehen ist, sowie ein Verfahren zur Einstellung eines Drehmoment-Gleich-gewichtszustandes einer an einer Haltevorrichtung drehbar gelagerten Masseeinheit.

Unter einer einstellbaren Kopplungseinheit wird insbesondere eine Kopplungseinheit verstanden, die es in einstellbarer Weise ermöglicht, zwischen der Wandlereinheit und der Masseeinheit einen Drehmomentschluß herzustellen bzw. die Wandlereinheit und die Masseeinheit bzgl. einer Drehmomentübertragung voneinander zu trennen. Bei einer solchen einstellbaren Kopplungseinheit kann es sich aber auch um eine Kopplungseinheit handeln, die zusätzlich oder alternativ zur Möglichkeit der Trennung einer Drehmomentübertragung von Wandlereinheit und Masseeinheit eine Übertragung eines von der Wandlereinheit bereitgestellten Drehmomentes auf die Masseeinheit ermöglicht, wobei mit der Kopplungseinheit eine Einstellung der Phasenlage des bereitgestellten Drehmomentes und die Übertragung des auf die Masseeinheit übertragenen Drehmomentes vorgenommen werden kann.

Eine derartige Vorrichtung und ein derartiges Verfahren sind aus der DE 37 390 80 A1 bekannt. Dort ist eine Federvorrichtung zum Gewichtsausgleich für Stative beschrieben, bei der mittels einer Linearfeder ein Drehmoment erzeugt wird, das dazu dient, das von einer Last in der Achse eines Schwenkarms hervorgerufene Last-Drehmoment zu kompensieren. Um einen Ausgleich des am Schwenkarm anliegenden Last-Drehmoments zu ermöglichen kann die Last am Schwenkarm in radialer Richtung verlagert werden oder der Angriffspunkt für die Linearfeder radial zur Schwenkachse verschoben werden.

In der DE 34 44 313 A1 ist eine Aufhängung für ein Operationsmikroskop beschrieben, bei der mittels einer Linearfeder ein kompensierendes Drehmoment für ein durch das Operationsmikroskop hervorgerufenes Last-Drehmoment erzeugt wird. Die Linearfeder wirkt dabei über einen Seilzug auf ein drehfest mit der Drehachse verbundenes Schneckenrad. Durch Verstellmechanismen an dem Schneckenrad kann einerseits die Amplitude des mittels der Linearfeder erzeugten kompensierenden Drehmoments und dessen Phasenlage bezüglich des Last-Drehmoments eingestellt werden.

Aus der DE 42 31 516 A1 ist es bekannt, zum Ausgleich des Lastmomentes, das von einem an einer Drehachse aufgehängten Operationsmikroskop hervorgerufen wird, eine Spiralfeder vorzusehen, die mittels einer Verstellschraube vorgespannt wird.

In der DE 39 21 857 A1 ist beschrieben, bei Mikroskopstativen zur Bereitstellung von Rückhaltekräften Gasdruckfedern in einem Gelenkparallelogramm einzusetzen.

Aufgabe der Erfindung ist es, eine Vorrichtung zum wenigstens teilweisen Ausgleich eines Last-Drehmoments zu schaffen, welche eine nahezu kräftefreie Bewegung einer Masseeinheit, insbesondere eines Operationsmikroskops, um eine Drehachse ermöglicht, deren Masseschwerpunkt sich in Abstand von dieser Drehachse befindet, sowie ein Verfahren bereitzustellen, das auf einfache Weise ermöglicht, für die Masseeinheit an der Drehachse einen Kräftegleichgewichtszustand einzustellen.

Diese Aufgabe wird durch eine Vorrichtung mit den Merkmalen des Anspruchs 1 und ein Verfahren mit den Merkmalen des Anspruchs 8 gelöst.

Auf diese Weise wird eine Vorrichtung geschaffen, welche das automatische Einstellen einer richtigen Phasenlage eines Kompensations-Drehmomentes, das mittels der Einheit zur Erzeugung von Linearkraft und der Wandlereinheit erzeugt wird, zu einem Last-Drehmoment ermöglicht, welches auf die Gewichtskraft zurückgeht, die am Schwerpunkt der an der Haltevorrichtung drehbar gelagerten Masseeinheit angreift. Der Kurbelmechanismus ermöglicht eine reibungsarme Wandlung einer Linearkraft in ein Drehmoment. Aufgrund der Einstellbarkeit der Länge des Kurbelarmes, der mit der Einheit zur Erzeugung von Linearkraft in Wirkverbindung steht, kann die Amplitude eines mittels der Einheit zur Erzeugung von Linearkraft generierten Kompensations-Drehmoments an ein auszugleichendes Last-Drehmoment angepasst werden.

In Weiterbildung der Erfindung ist zur Einstellung der Länge des Kurbelarmes eine Gewindespindel vorgesehen. Auf diese Weise ist es möglich, die Amplitude eines Kompensations-Drehmoments präzise einzustellen.

In Weiterbildung der Erfindung umfasst die einstellbare Kopplungseinheit ein Schneckengetriebe. Auf diese Weise ist es möglich, die Phase eines Kompensations-Drehmoments präzise einzustellen.

In Weiterbildung der Erfindung umfasst die einstellbare Kopplungseinheit einen Rastmechanismus. Auf diese Weise können von der Wandlereinheit auf die Masseeinheit große Drehmomente übertragen werden.

In Weiterbildung der Erfindung weist der Rastmechanismus ein Rastrad auf, das Bohrungen zum Eingriff eines Raststiftes aufweist. Auf diese Weise wird ein verschleißarmer Kopplungsmechanismus von Wandlereinheit und Masseeinheit bereitgestellt.

In Weiterbildung der Erfindung ist dem Raststift ein Halteglied zugeordnet, welches in einer Führungsnut geführt ist, wobei durch Bewegen des Haltegliedes in der Führungsnut die Masseeinheit und die Wandlereinheit aneinander gekoppelt und voneinander entkoppelt werden können. Auf diese Weise kann die Kopplungseinheit über das Einstellen eines in der Wandlereinheit wirkenden Kurbelarmes betätigt werden.

In Weiterbildung der Erfindung ist die Einheit zur Erzeugung von Linearkraft als Gasdruckfeder ausgebildet. Auf diese Weise kann eine Vorrichtung zum wenigstens teilweisen Ausgleich eines Last-Drehmoments mit geringem Eigengewicht geschaffen werden, in der zur Erzeugung eines Kompensations-Drehmoments große Linearkräfte bereitgestellt werden.

Bei einem Verfahren zur Einstellung eines Drehmoment-Geleichgewichtszustandes für eine an einer Haltevorrichtung drehbar gelagerte Masseeinheit gemäß Anspruch 8 wird die Masseeinheit von einer Wandlereinheit zur Wandlung einer Linearkraft in ein Drehmoment entkoppelt und nimmt die Masseeinheit unter Einwirkung der Gewichtskraft und die Wandlereinheit unter Einwirkung einer Einheit zur Erzeugung einer Linearkraft einen stabilen Gleichgewichtszustand ein. Dann wird in dem jeweiligen stabilen Gleichgewichtszustand die Masseeinheit mit der Wandlereinheit gekoppelt. Auf diese Weise ist es möglich, schnell einen Drehmoment-Ausgleichszustand für ein an einer Haltevorrichtung drehbar gelagertes Operationsmikroskop einzustellen.

In Weiterbildung des Verfahrens wird nach Kopplung von Masseeinheit und Wandlereinheit die Länge eines Kurbelarmes in der Wandlereinheit eingestellt. Auf diese Weise wird ein Feinabgleich von Kompensations-Drehmoment und Last-Drehmoment an einem Operationsmikroskop ermöglicht.

Weitere Merkmale und Vorteile der Erfindung sind in den Zeichnungen dargestellt und werden nachfolgend beschrieben.

Es zeigen:

Fig. 1: eine Perspektivansicht einer ersten Ausführungsform einer Vorrichtung zum wenigstens teilweisen Ausgleich eines Last-Drehmoments;

Fig. 2: ein an einer Drehachse aufgenommenes Operationsmikroskop in Seitenansicht;

Fig. 3: eine Phasenlage von Last-Drehmoment und Kompensation-Drehmoment für Drehmoment-Ausgleich; und

Fig. 4: eine Perspektivansicht einer zweiten Ausführungsform einer Vorrichtung zum wenigstens teilweisen Ausgleich eines Last-Drehmoments.

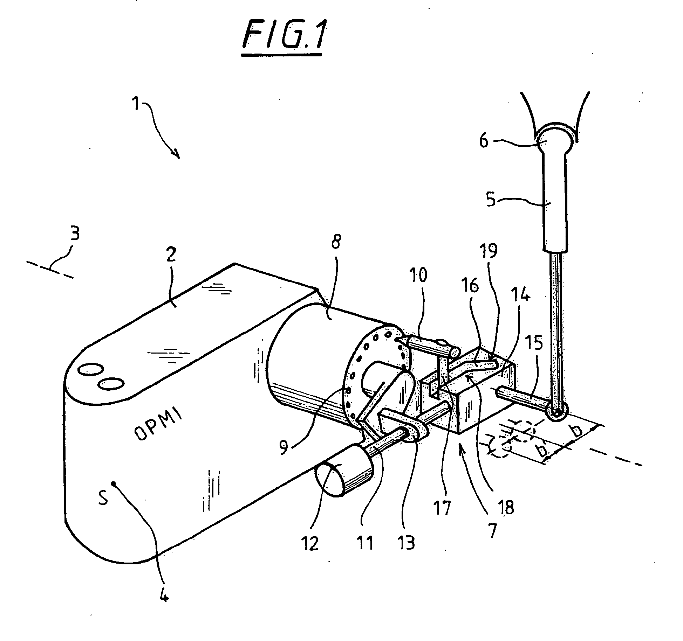

Die Vorrichtung 1 zum wenigstens teilweisen Ausgleich eines Last-Drehmoments aus Fig. 1 umfasst ein Operationsmikroskop 2, welches als Masseeinheit auf einer Drehachse 3 mit einer nicht weiter dargestellten Freilauflagereinheit gelagert ist. Das Operationsmikroskop 2 hat einen außerhalb der Drehachse 3 liegenden Masseschwerpunkt 4. Die Lage des Masseschwerpunktes 4 wird durch den Aufbau des Operationsmikroskopes 2 bestimmt. Diese ändert sich in der Regel durch den Anschluß von Zusatzgeräten, wie beispielsweise Mitbeobachtungseinrichtungen oder Kameras. Durch die im Massenschwerpunkt 4 des Operationsmikroskopes 2 angreifende Gewichtskraft entsteht ein Last-Drehmoment, welches bestrebt ist, das Operationsmikroskop 2 in eine stabile Gleichgewichtsstellung zu bewegen. Dieser stabilen Gleichgewichtsstellung entspricht eine Position des Massenschwerpunktes 4 von Operationsmikroskop 2 unterhalb der Drehachse 3 auf dem zur Drehachse 3 senkrechten Lot. In dieser Stellung des Operationsmikroskopes 2 ruft die am Gerät angreifende Gewichtskraft kein Last-Drehmoment hervor.

Zum Ausgleich dieses Last-Drehmoments ist in der Vorrichtung 1 eine Gasdruckfeder 5 vorgesehen. Anstelle einer Gasdruckfeder 5 kann jedoch genausogut eine Gaszugfeder, eine Zug- oder Druckfeder etwa in Form einer Schraubenfeder oder ein entsprechender anders ausgebildeter Energiespeicher eingesetzt werden, der sich für die Bereitstellung einer Linearkraft eignet.

Die Gasdruckfeder 5 ist mit einem Kugelgelenk 6 an einer nicht weiter dargestellten Stativeinheit drehbar gelagert. Sie ist mit einer Wandlereinheit 7 verbunden, welche eine von der Gasdruckfeder 5 erzeugte Linearkraft in ein Kompensations-Drehmoment wandelt. Mit diesem Kompensations-Drehmoment wird das an dem Operationsmikroskop 2 auftretende Last-Drehmoment ausgeglichen. Die Wandlereinheit 7 ist als Kurbelmechanismus ausgebildet. Dieser Kurbelmechanismus umfaßt einen an einer Gewindespindel 11 aufgenommenen Nutenstein 14. An diesem Nutenstein 14 greift mit einem Anschlußarm 15 die Gasdruckfeder 5 an. Die Gewindespindel 11 und der Nutenstein 14 wirken damit als Kurbelarm, der unter Einwirkung der Linearkraft der Gasdruckfeder 5 bestrebt ist, eine Drehbewegung um die Drehachse 3 auszuführen.

Zur Kopplung des Kurbelarmes an das Operationsmikroskop 2 ist eine einstellbare Kopplungseinheit vorgesehen. Diese Kopplungseinheit weist einen Rastmechanismus auf, die ein mit dem Operationsmikroskop 2 drehfest verbundenes Rastrad 8 mit Bohrungen 9 umfaßt. In eine der Bohrungen 9 greift ein Raststift 10 ein. Mittels des Raststiftes 10 wird ein mit der Gasdruckfeder 5 generiertes Kompensations-Drehmoment von der Wandlereinheit 7 auf das Operationsmikroskop 2 übertragen. Die Wirklänge des Kurbelarmes in der Wandlereinheit 7 kann durch Drehen der Gewindespindel 11 eingestellt werden. Hierfür hat die Gewindespindel 11 einen Drehknopf 12, mit dem sie in einer Gewindebohrung 13 gedreht werden kann. Durch das Drehen der Gewindespindel 11 in der Gewindebohrung 13 wird die Position des Nutensteines 14 und damit die Wirklänge des Kurbelarmes, auf den die Gasdruckfeder 5 über den Anschlußarm 15 mit ihrer Federkraft wirkt, verändert.

Der Raststift 10 ist an einem Halteglied 17 aufgenommen, das über die Gewindespindel 11 in einer Mimik mit Führungsnut 16 im Nutenstein 14 geführt ist. Durch Drehen der Gewindespindel 11 kann der Nutenstein 14 senkrecht zur Drehachse 3 hin- und herbewegt werden. Mit der Bewegung des Nutensteines 14 verändert sich die Lage des Haltegliedes in der Führungsnut 16. Entsprechend der Stellung des Nutensteines 14 ändert sich so die Wirklänge des Kurbelarmes, der mit der Gasdruckfeder 5 beaufschlagt ist, und es ergeben sich unterschiedliche Kompensations-Drehmomente.

Die Führungsnut 16 in dem Nutenstein 14 hat einen ersten Abschnitt 18, in dem die Führungsnut 16 senkrecht zur Drehachse 3 verläuft. In einem zweiten Abschnitt 19 ist die Führungsnut 16 entsprechend einem Verlauf mit Winkel zur Drehachse 3 ausgebildet. Mit einem Verfahren des Nutensteines 14 wird das Halteglied 17 mit dem daran angeordneten Raststift 10 in dem Bereich der Führungsnut 16 in Richtung der Drehachse 3 bewegt. Damit gelangt der Raststift 10 mit den Bohrungen 9 in dem Rastrad 8 in Eingriff bzw. wird dieser von dem Rastrad 8 entkoppelt. Mit einem Verfahren des Nutensteines 14 und die entsprechende Wahl des Loches im Rastrad 8, in das der Raststift 10 zum Eingriff gebracht wird, kann ein Mittels der Gasdruckfeder 5 erzeugtes Kompensations-Drehmoment bezüglich Phase und Amplitude an ein gegebenes Last-Drehmoment angepasst werden.

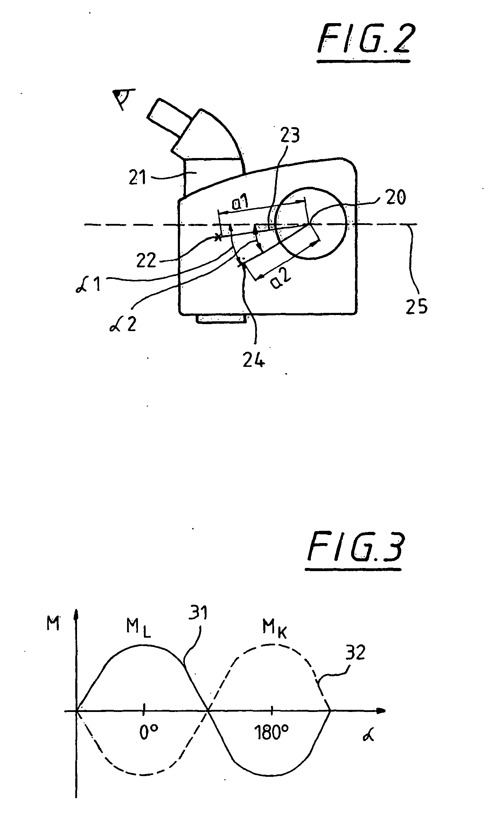

Fig. 2 erläutert den Zusammenhang zwischen einer Schwerpunktverlagerung und der entsprechenden Änderung des Last-Drehmomentes bei einem an einer Drehachse 20 aufgenommenen Operationsmikroskop 21, das in Seitenansicht gezeigt ist. Das Operationsmikroskop 21 hat einen Masseschwerpunkt mit Position 22. Die an dem Massenschwerpunkt angreifende Gewichtskraft wirkt mit einem Hebelarm 23 der Länge $a_1$ auf die Drehachse 20 und bewirkt dort ein Last-Drehmoment $M_{L_1}$. Durch anbringen von Zusatzgerät, beispielsweise einer Videokamera, Zusatzlinsen oder Vorsatzmodule verlagert sich dieser Masseschwerpunkt von der Position 22 zur Position 24. Die Änderung der Gesamtmasse des Operationsmikroskop-Systems und die Verlagerung des Masseschwerpunktes führt zu einer entsprechenden Änderung des Last-Drehmoments.

Beim Operationsmikrosop 21 aus Fig. 2 gilt für das Last-Drehmoment $M_{L_1}$ des Systems bei einer Position 22 für den Masseschwerpunkt:

$$M_{L_1} = m_1 g a_1 \cos(\alpha_1),$$

wobei:

$m_1$ die Gesamtmasse des Operationsmirkoskops bei einem Masseschwerpunkt mit der Position 22,

$g$ die Konstante der Erdbeschleunigung,

$a_1$ der Abstand des Operationsmikroskop-Masseschwerpunkts mit Position 22 von der Drehachse 20, und

$\alpha_1$ der Winkel zwischen der Verbindungslinie von Masseschwerpunkt und Drehachse 20 zur Horizontalen 25 ist.

Mit Anschluß von Zusatzgeräten ändert sich die Gesamtmasse des Operationsmikroskopes von $m_1$ auf $m_2$. Gleichzeitig verlagert sich der Masseschwerpunkt des Operationsmikroskops von der Position 22 zur Position 24. Für das Last-Drehmoment $M_{L_2}$ ergibt sich hier:

$$M_{L_2} = m_2 g a_2 \cos(\alpha_2),$$

wobei:

$m_2$ die Gesamtmasse des Operationsmirkoskops bei einem Masseschwerpunkt mit der Position 24,

$g$ die Konstante der Erdbeschleunigung,

$a_2$ der Abstand des Operationsmikroskop-Masseschwerpunkts mit Position 24 von der Drehachse 20, und

$\alpha_2$ der Winkel zwischen der Verbindungslinie von Masseschwerpunkt und Drehachse 20 zur Horizontalen 23 ist.

Eine Änderung der Gesamtmasse des Operationsmikroskops und die Verlagerung von dessen Schwerpunkt verändert damit in Allgemeinen das an der Drehachse 20 auftretende Last-Drehmoment. Um eine kräftefreie Bewegung des Operationsmikroskopsystems um die Drehachse 20 zu ermöglichen, ist eine entsprechende Anpassung von Phase und Amplitude des Kompensations-Drehmoments erforderlich.

Die Figur 3 zeigt mit der Kurve 31 die Abhängigkeit eines Last-Drehmoments $M_L$ in Abhängigkeit der Winkelstellung $\alpha$ eines Operationsmikroskopes auf einer Drehachse. Für eine vollständige Kompensation diese Last-Drehmoments muß ein Kompensations-Drehmoment $M_K$ bereitgestellt werden, dessen Phasenlage und Amplitude an das Last-Drehmoment $M_L$ derart angepasst ist, dass sich Kompensations-Drehmoment $M_K$ und Last-Drehmoment $M_L$ gegenseitig vollständig aufheben. Hat bei einem Operationsmikroskop ein Kompenations-Drehmoment $M_K$ einen der Kurve 32 entsprechenden Verlauf, so befindet sich das Operationsmikroskop in jeder Winkelstellung bezüglich einer der Drehachse, mit der es aufgenommen ist, im Kräftegleichgewicht. Somit ist es möglich, das Operationsmikroskop um diese Drehachse nahezu kräftefrei zu bewegen.

Nachfolgend wird anhand von Fig. 1 beschrieben, wie bei gegebenem Last-Drehmoment $M_L$ ein passendes Kompensations-Drehmoment $M_K$ eingestellt werden kann. Zunächst wird hierfür durch Öffnen des Rastmechanismus das Operationsmikroskop 2 von der Wandlereinheit 7 und der Gasdruckfeder 5 entkoppelt. In Folge dessen kippt unter Einwirkung der Schwerkraft das Operationsmikroskop 2 um die Drehachse 3 in eine stabile Gleichgewichtslage. In dieser stabilen Gleichgewichtslage befindet sich der Masseschwerpunkt 4 des Systems mit der Drehachse 3 im Lot. Gleichzeitig wird dann mittels der Gasdruckfeder 5 die Wandlereinheit 7 in eine stabile Gleichgewichtslage getrieben, in welcher der Federarm der Gasdruckfeder 5 und der Kurbelarm der Wandlereinheit 7 aus Nutenstein 14 und Gewindespindel 11 parallel zueinander ausgerichtet sind. Wird in diesen einander entsprechenden stabilen Gleichgewichtslagen von Operationsmikroskop 2 und der Wandlereinheit 7 mit Gasdruckfeder 5 die Kupplungseinheit geschlossen, so haben das von Operationsmikroskop 2 hervorgerufen Last-Drehmoment $M_L$ und das mit der Gasdruckfeder 5 erzeugte Kompensations-Drehmoment $M_K$ eine zueinander um 180° verschobene Phasenlage. Indem dann durch Verdrehen der Gewindespindel 11 die Wirklänge eines Kurbelarmes in der Wandlereinheit 7 eingestellt wird, kann die Amplitude dieses Kompensations-Drehmoments $M_K$ an die Amplitude des Last-Drehmoments $M_L$ angepasst werden. Die Folge ist ein vollständiger Drehmomentausgleich für das an der Drehachse 3 aufgenomme Operationsmikroskop 4 in einer jeden Winkelstellung.

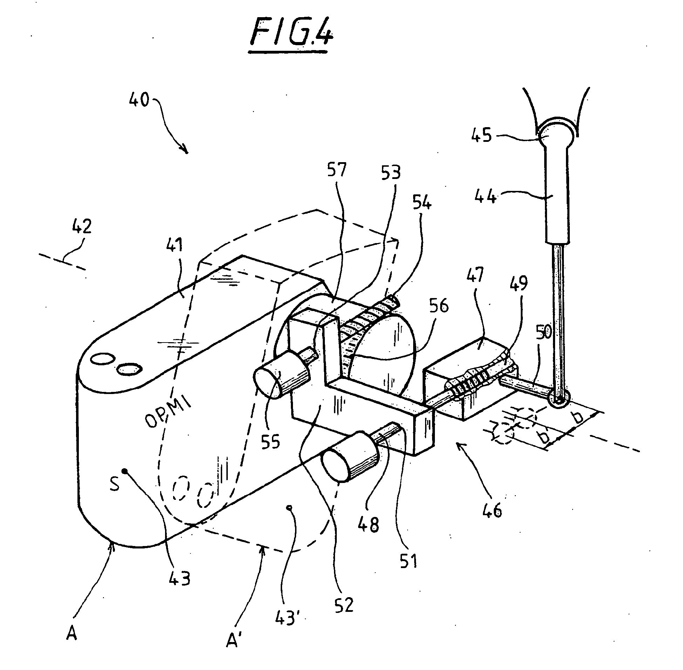

Die Fig. 4 zeigt eine Vorrichtung 40 zum wenigstens teilweisen Ausgleich eines Last-Drehmoments in alternativer Ausführungsform zu der Vorrichtung aus Fig. 1. Die Vorrichtung 40 umfasst ein Operationsmikroskop 41, welches als Masseeinheit auf einer Drehachse 42 mit einer nicht weiter dargestellten Freilauflagereinheit gelagert ist. Das Operationsmikroskop 41 hat in einer Stellung A einen Massenschwerpunkt, der außerhalb der Drehachse 42 am Ort 43 liegt. Wie bei der Vorrichtung 1 aus Fig. 1 wird bei der Vorrichtung 40 die Lage des Masseschwerpunktes durch den Aufbau des Operationsmikroskopes 41 bestimmt. Durch die im Massenschwerpunkt des Operationsmikroskopes 41 angreifende Gewichtskraft entsteht ein Last-Drehmoment, welches bestrebt ist, das Operationsmikroskop 41 aus der Stellung A in eine stabile Gleichgewichtsstellung zu bewegen. Dieser stabilen Gleichgewichtsstellung entspricht eine Stellung A' des Operationsmikroskopes mit einer Position 43' des Massenschwerpunktes unterhalb der Drehachse 42 auf dem zur Drehachse 42 senkrechten Lot. In dieser Stellung des Operationsmikroskopes 41 ruft die am Gerät angreifende Gewichtskraft kein Last-Drehmoment hervor.

Zum Ausgleich dieses Last-Drehmoments ist in der Vorrichtung 40 eine Gasdruckfeder 44 vorgesehen, die jedoch genauso gut als Gaszugfeder, als Zug- oder Druckfeder in Form einer Schraubenfeder oder irgend ein entsprechend anderer Energiespeicher, der sich für die Bereitstellung einer Linearkraft eignet, ausgebildet sein kann.

Die Gasdruckfeder 44 ist mit einem Kugelgelenk 45 an einer nicht weiter dargestellten Stativeinheit drehbar gelagert. Sie ist mit einer als Kurbelmechanismus ausgebildeten Wandlereinheit 46 verbunden, welche eine von der Gasdruckfeder 44 erzeugte Linearkraft in ein Kompensations-Drehmoment wandelt. Wie bei der Wandlereinheit 7 aus Fig. 1 umfaßt dieser Kurbelmechanismus einen Kurbelblock 47 mit einer Gewindespindel 48, die im Kurbelblock 47 in einem Schraubgewinde 49 geführt ist. An dem Kurbelblock 47 greift mit einem Anschlußarm 50 die Gasdruckfeder 44 an. Die Gewindespindel 48 hat einen Drehknopf und ist mit einer Lagerstelle 51 in einem Aufnahmeteil 52 drehbar gelagert.

Die Gewindespindel 48 und der Kurbelblock 47 wirken damit als Kurbelarm, der unter Einwirkung der Linearkraft der Gasdruckfeder 44 bestrebt ist, eine Drehbewegung um die Drehachse 42 auszuführen. Durch Drehen der Gewindespindel 48 kann die Länge des Kurbelarmes eingestellt werden.

Das mit der Wandlereinheit bereitgestellte Kompensations-Drehmoment wird über eine einstellbare Kopplungseinheit in Form eines Schneckengetriebes in das Operationsmikroskop 41 geleitet, um ein dort auftretendes Last-Drehmoment auszugleichen. Hierfür ist in einer weiteren Lagerstelle 53 im Aufnahmeteil 52 eine Schnecke 54 aufgenommen, die über einen Drehknopf 55 gedreht werden kann. Diese Schnecke 54 greift in einen Schneckenzahnkranz 56, der auf einem mit dem Operationsmikroskop 41 drehfest verbundenen Anschlußrad 57 vorgesehen ist. Dieses Anschlußrad 57 leitet ein mittels der Gasdruckfeder 44 und dem Kurbelblock 47 mit Gewindespindel 48 erzeugtes Kompensations-Drehmoment in das Operationsmikroskop 41. Durch Drehen der Schnecke 54 kann das Operationsmikroskop 41 um die Drehachse 42 gekippt werden. Es ist damit möglich, die Phasenlage eines Kompensations-Drehmomentes zu einem am Operationsmikroskop 41 anliegenden Last-Drehmoment präzise einzustellen.

**Patentansprüche**

1. Vorrichtung (1) zum wenigstens teilweisen Ausgleich eines Last-Drehmoments, welches an einer Masseeinheit, insbesondere einem Operationsmikroskop (2), angreift, die an einer Haltevorrichtung drehbar gelagert ist, mit

   - einer Einheit zur Erzeugung von Linearkraft (5, 44) und
   - einer Wandlereinheit (7, 46), welche die Linearkraft in ein Drehmoment wandelt,
   - einer einstellbaren Kopplungseinheit (8, 9, 10, 54, 55, 56) zur Übertragung des Drehmoments zwischen der Wandlereinheit (7, 46) und der Masseeinheit (2, 41),

   **dadurch gekennzeichnet, dass**

   - die Wandlereinheit (7, 46) als Kurbelmechanismus ausgebildet ist, wobei
   - in der Wandlereinheit (7, 46) die Länge (b, b') eines Kurbelarmes, der mit der Einheit zur Erzeugung von Linearkraft (5, 44) in Wirkverbindung steht, einstellbar ist.

**2.** Vorrichtung gemäß Anspruch 1, **dadurch gekennzeichnet, dass** zur Einstellung der Länge des Kurbelarmes eine Gewindespindel (11, 48) vorgesehen ist.

**3.** Vorrichtung gemäß Anspruch 1 oder 2, **dadurch gekennzeichnet, dass** die einstellbare Kopplungseinheit (54, 55, 56) ein Schneckengetriebe umfasst.

**4.** Vorrichtung gemäß einem der Ansprüche 1-3, **dadurch gekennzeichnet, dass** die Kopplungseinheit (8, 9, 10) einen Rastmechanismus umfasst.

**5.** Vorrichtung gemäß Anspruch 4, **dadurch gekennzeichnet, dass** der Rastmechanismus ein Rastrad (8) aufweist, in dem Bohrungen (9) zum Eingriff eines Raststiftes (10) vorgesehen sind.

**6.** Vorrichtung gemäß Anspruch 5, **dadurch gekennzeichnet, dass** dem Raststift (10) ein Haltglied (17) zugeordnet ist, welches in einer Führungsnut (16) geführt ist, wobei durch Bewegen des Hteglliedes (17) in der Führungsnut (16) die Masseeinheit (2) und die Wandlereinheit (7) aneinander gekoppelt und voneinander entkoppelt werden können.

**7.** Vorrichtung gemäß einem der Ansprüche 1-6 **dadurch gekennzeichnet, dass** die Einheit zur Erzeugung von Linearkraft als Gasdruckfeder (5) oder als Gaszugfeder ausgebildet ist.

**8.** Verfahren zur Einstellung eines Drehmoment-Gleichgewichtszustandes einer an einer Haltevorrichtung drehbar gelagerten Masseeinheit, insbesondere einer Vorrichtung gemäß einem der Ansprüche 1, 2 oder -4-7
**dadurch gekennzeichnet, dass**

- die Masseeinheit (2) von einer Wandlereinheit (7) zur Wandlung einer Linearkraft in ein Drehmoment entkoppelt wird,
- die Masseeinheit (2) unter Einwirkung der Gewichtskraft einen stabilen Gleichgewichtszustand einnimmt,
- die Wandlereinheit (7) unter Einwirkung einer Einheit zur Erzeugung einer Linearkraft (5) einen stabilen Gleichgewichtszustand einnimmt, und
- in dem jeweiligen stabilen Gleichgewichtszustand die Masseeinheit (2) mit der Wandlereinheit (7) gekoppelt wird.

**9.** Verfahren gemäß Anspruch 8, **dadurch gekennzeichnet, dass** nach Kopplung von Masseeinheit (2) und Wandlereinheit (7) die Länge eines Kurbelarmes in der Wandlereinheit (7) eingestellt wird.

**Claims**

**1.** Apparatus (1) for at least partially equalizing a load torque, which apparatus acts upon a mass unit, in particular an operating microscope (2), rotatably mounted on a holding apparatus, comprising

- a unit for generating linear force (5, 44), and
- a converter unit (7, 46), which converts the linear force into a torque,
- an adjustable coupling unit (8, 9, 10, 54, 55, 56) for transmitting the torque between the converter unit (7, 46) and the mass unit (2, 41),

**characterized in that**

- the converter unit (7, 46) is configured as a crank mechanism,
- in the converter unit (7, 46), the length (b, b') of a crank arm operatively connected to the unit for generating linear force (5, 44) being adjustable.

**2.** Apparatus according to Claim 1, **characterized in that** a threaded spindle (11, 48) is provided for adjusting the length of the crank arm.

**3.** Apparatus according to Claim 1 or 2, **characterized in that** the adjustable coupling unit (54, 55, 56) comprises a worm gear.

**4.** Apparatus according to one of Claims 1-3, **characterized in that** the coupling unit (8, 9, 10) comprises a latching mechanism.

**5.** Apparatus according to Claim 4, **characterized in that** the latching mechanism has a ratchet wheel (8), in which bores (9) for the engagement of a latch pin (10) are provided.

**6.** Apparatus according to Claim 5, **characterized in that** to the latch pin (10) there is assigned a holding member (17) which is guided in a guide groove (16), the mass unit (2) and the converter unit (7) being able to be coupled to one another and decoupled from one another by movement of the holding member (17) in the guide groove (16).

**7.** Apparatus according to one of Claims 1-6, **characterized in that** the unit for generating linear force is configured as a gas compression spring (5) or as a gas tension spring.

**8.** Method for adjusting a torque state of equilibrium of a mass unit rotatably mounted on a holding apparatus, especially of an apparatus according to one of Claims 1, 2 or 4-7,
**characterized in that**

- the mass unit (2) is decoupled from a converter unit (7) for converting a linear force into a torque,
- the mass unit (2), under the effect of the weight, assumes a stable state of equilibrium,
- the converter unit (7), under the effect of a unit for generating a linear force (5), assumes a stable state of equilibrium, and
- in the respective stable state of equilibrium, the mass unit (2) is coupled to the converter unit (7).

9. Method according to Claim 8, **characterized in that**, following coupling of the mass unit (2) and the converter unit (7), the length of a crank arm in the converter unit (7) is adjusted.

**Revendications**

1. Dispositif (1) pour équilibrer au moins partiellement un couple de charge, qui agit sur une unité de masse, en particulier un microscope opérationnel (2), qui est monté de façon rotative sur un dispositif de support, avec

- une unité de production d'une force linéaire (5, 44),
- une unité de conversion (7, 46), qui convertit la force linéaire en un couple, et
- une unité de couplage réglable (8, 9, 10, 54, 55, 56) pour transmettre le couple entre l'unité de conversion (7, 46) et l'unité de masse (2, 41),

**caractérisé en ce que**

- l'unité de conversion (7, 46) est réalisée sous la forme d'un mécanisme à manivelle, dans lequel
- dans l'unité de conversion (7, 46), la longueur (b, b') d'un bras de manivelle, qui est en liaison active avec l'unité de production d'une force linéaire (5, 44), est réglable.

2. Dispositif selon la revendication 1, **caractérisé en ce qu'**il est prévu une broche filetée (11, 48) pour le réglage de la longueur du bras de manivelle.

3. Dispositif selon la revendication 1 ou 2, **caractérisé en ce que** l'unité de couplage réglable (54, 55, 56) comprend un engrenage à vis sans fin.

4. Dispositif selon l'une quelconque des revendications 1 à 3, **caractérisé en ce que** l'unité de couplage (8, 9, 10) comprend un mécanisme à encliquetage.

5. Dispositif selon la revendication 4, **caractérisé en ce que** le mécanisme à encliquetage comprend une roue d'arrêt (8), dans laquelle il est prévu des alésages (9) pour l'engagement d'une tige d'arrêt (10).

6. Dispositif selon la revendication 5, **caractérisé en ce qu'**un organe de support (17) est associé à la tige d'arrêt (10), lequel est guidé dans une rainure de guidage (16), dans lequel l'unité de masse (2) et l'unité de conversion (7) sont couplées l'une à l'autre et séparées l'une de l'autre par un déplacement de l'organe de support (17) dans la rainure de guidage (16).

7. Dispositif selon l'une quelconque des revendications 1 à 6, **caractérisé en ce que** l'unité de production d'une force linéaire est réalisée sous la forme d'un vérin de poussée à gaz (5) ou d'un vérin de traction à gaz.

8. Procédé pour régler un état d'équilibre de couple d'une unité de masse montée de façon rotative sur un dispositif de support, en particulier d'un dispositif selon l'une quelconque des revendications 1, 2 ou 4 - 7,
**caractérisé en ce que**

- on sépare l'unité de masse (2) d'une unité de conversion (7) pour convertir une force linéaire en un couple,
- l'unité de masse (2) atteint un état d'équilibre stable sous l'action de son poids,
- l'unité de conversion (7) atteint un état d'équilibre stable sous l'action d'une unité de production d'une force linéaire (5), et
- on couple l'unité de masse (2) avec l'unité de conversion (7) dans l'état d'équilibre stable respectif.

9. Procédé selon la revendication 8, **caractérisé en ce qu'**après le couplage de l'unité de masse (2) avec l'unité de conversion (7), on règle la longueur d'un bras de manivelle dans l'unité de conversion (7).

# FIG.1

## _FIG.2_

## _FIG.3_

# FIG.4